(19) Europäisches Patentamt
European Patent Office
Office européen des brevets

(11) Publication number: **0 171 249 A2**

# (12) EUROPEAN PATENT APPLICATION

(21) Application number: **85305401.3**

(22) Date of filing: **29.07.85**

(51) Int. Cl.⁴: **C 07 C 49/577**, C 07 C 49/813, C 07 C 45/00, A 01 N 35/02, A 01 N 35/04

(30) Priority: **30.07.84 GB 8419339**

(43) Date of publication of application: **12.02.86 Bulletin 86/7**

(84) Designated Contracting States: **CH DE FR IT LI NL**

(71) Applicant: **NATIONAL RESEARCH DEVELOPMENT CORPORATION, 101 Newington Causeway, London SE1 6BU (GB)**

(72) Inventor: **Elliott, Michael, 9 Long Ridge, Aston Stevenage Herts. SG2 7EW (GB)**
Inventor: **Janes, Norman Frank, 82 Marston Gardens, Luton Beds. LU2 7DY (GB)**
Inventor: **Khambay, Bhupinder Pall Singh, 85 Warham Road, Harrow Weald Middx. HA3 7JA (GB)**

(74) Representative: **Cardnell, Peter Harry Morley, Patent Department National Research Development Corporation 101 Newington Causeway, London SE1 6BU (GB)**

## (54) Improvements relating to pesticides.

(57) Pesticidal compounds of formula I, the preparation of such compounds, intermediates for use in their preparation compositions containing such compounds and the use of such compounds and compositions to prevent or control pest infestation. Compounds of I have the following formula:

I $R_ACOCHDR_B$

in which formula:

$R_A$ represents a group $ArCR_1R_2$- in which Ar represents a phenyl or naphthyl group optionally substituted by one or more halogen, haloalkoxy, methylenedioxy or $C_1$-$C_6$ alkyl groups;

$R_1$ representing methyl, ethyl, isopropyl or cycloalkyl;

$R_2$ representing hydrogenn or methyl; or

$R_1$ and $R_2$ together with the carbon to which they are attached representing jointly a cycloalkyl group optionally carrying one or more halogen substituents;

or $R_A$ represents the residue of a pyrethroidal acid $R_ACO_2H$ the 5-benzyl-3-furylmethyl ester of which is significantly insecticidal;

$R_B$ represents the residue of an alcohol $R_BCHDOH$ in which D is hydrogen or cyano and of which the [IR, cis] 2,2-dimethyl-3-(2,2-dibromovinyl)cyclopropane carboxylic ester is significantly insecticidal, provided that then the alcohol is of formula II:

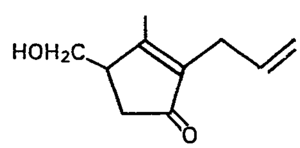

$R_A$ is other than the residue of chrysanthemic acid.

EP 0 171 249 A2

J27434

# IMPROVEMENTS RELATING TO PESTICIDES

This invention relates to pesticides and in particular to pesticidal compounds, the preparation of such compounds, intermediates for use in their preparation, compositions containing such compounds and the use of such compounds and compositions to prevent or control pest infestation.

Accordingly the present invention comprises a compound of formula I

I $R_ACOCHDR_B$

in which formula:-

$R_A$ represents a group $ArCR_1R_2$- in which Ar represents a phenyl or naphthyl group optionally substituted by one or more halogen, haloalkoxy, methylenedioxy or $C_1$-$C_6$ alkyl groups;

$R_1$ representing methyl, ethyl, isopropyl or cycloalkyl;

$R_2$ representing hydrogen or methyl; or

$R_1$ and $R_2$ together with the carbon to which they are attached representing jointly a cycloalkyl group optionally carrying one or more halogen substituents;

or $R_A$ represents the residue of a pyrethroidal acid $R_ACO_2H$ the 5-benzyl-3-furylmethyl ester of which is significantly insecticidal;

$R_B$ represents the residue of an alcohol $R_BCHDOH$ in which D is hydrogen or cyano and of which the [IR, cis] 2,2-dimethyl-3-(2,2-dibromovinyl)cyclopropane carboxylic ester is significantly insecticidal, provided that when the alcohol is of formula II

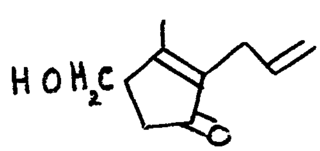

$R_A$ is other than the residue of chrysanthemic acid.

When $R_A$ represents $ArCR_1R_2$, Ar, which typically represents phenyl is usually substituted at the 3-(meta) and/or 4-(para)-position by chlorine, ethoxy or haloalkyl e.g. $CF_3$ or, a haloalkoxy group, which may comprise one or more halogens. $-OCF_3$,$-OCF_2H$ and $-OCFH_2$, are of particular interest. The substituents $R_1$ and $R_2$, together with the carbon bearing them, preferably represent a substituted or unsubstituted cyclopropyl group or $R_1$ and $R_2$ each represent methyl or $R_1$ represents isopropyl and $R_2$ hydrogen. In the former case, when the cyclopropyl group is substituted, the halogen is preferably fluorine. Cyclopropane groups carrying two fluorine atoms on the same or a different carbon are of especial interest. When fluorine is present at different carbons in the cyclopropane ring various stereoisomers are obviously possible and the present invention includes within its scope each such individual stereoisomer and mixtures thereof.

When the carbon carrying $R_1$ and $R_2$ is asymmetric the compound of formula I can of course exist in R & S configurations; both mixtures of isomers and single isomers are included within the scope of the present invention.

When $R_A$ represents the residue of a pyrethroidal acid $R_ACO_2H$, the acid is generally such that the 5-benzyl-3-furylmethyl ester thereof shows a potency of at least 5 and usually at least 10 relative to bioresmethrin = 100 towards houseflies.

There is a large number of pyrethroidal acids which are cyclopropane carboxylic acids in which $R_A$ is a group of the formula:-

II 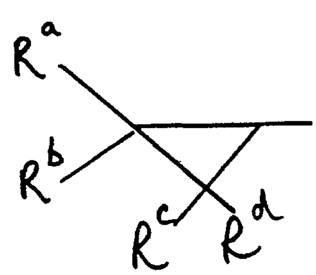

In formula II $R^c$ and $R^d$ will normally be an alkyl group, usually the same alkyl group, containing 1 to 4 carbon atoms and, as is

well known in the art, dimethyl substitution normally gives high activity.

$R^b$ in formula II is normally hydrogen or an alkyl group containing 1 to 4 carbon atoms and here the experience of the art indicates that $R^b$ will usually be hydrogen for maximum activity except in those compounds where $R^a$ is also an alkyl group, in which case $R^b$ preferably is an alkyl group, $R^a$, $R^b$, $R^c$ and $R^d$ all conveniently being the same alkyl group, e.g. methyl.

In formula II $R^a$ can be hydrogen or a substituted or unsubstituted acyclic or carbocyclic group. When $R^a$ is an unsubstituted hydrocarbyl group, it can be a straight chain or branched saturated or unsaturated acyclic or carbocyclic group such as an alkyl group, an alkenyl or alkadienyl group or a cycloalkyl, cycloalkylalkyl or cycloalkylalkenyl group. These hydrocarbyl groups preferably contain up to 10, particularly up to 6 carbon atoms.

When $R^a$ is substituted, it is preferably one of the hydrocarbyl groups mentioned above which is substituted by one or more halogeno groups which may be fluorine, chlorine or bromine or by an alkoxy or oximino group or alkoxycarbonyl group, as in group $R^a$ of particular interest of formula $-CH=CHCO_2R_x$ wherein $R_x$ represents an alkyl group typically containing 1 to 4 carbon atoms. When the substituents are two or more halogeno substituents, the halogeno substituents need not necessarily be the same halogen while when alkoxy groups are present, these preferably contain up to 4 carbon atoms and will normally be methoxy groups.

One particularly valuable structure for the group $R^a$ is of formula III:-

III

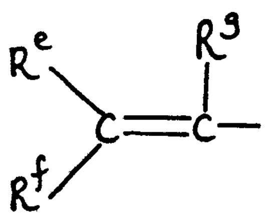

where $R^e$ and $R^f$, which may be the same or different, are each an alkyl group containing 1 to 4 carbon atoms, a trifluoromethyl group or a halogeno group, which may be the same or different and are preferably fluorine, chlorine or bromine. $R^g$ is a $C_1$-$C_4$ alkyl group or, preferably, hydrogen. One of $R^e$ and $R^f$ may also represent hydrogen or a phenyl or substituted phenyl group. Alternatively, $R^e$ and $R^f$ may together form a straight or branched substituted or unsubstituted saturated or unsaturated divalent hydrocarbon chain which may be substituted by one or more hetero atoms e.g. O, N or S, so that $R^e$ and $R^f$ together with the carbon atom to which they are attached form a carbocyclic or heterocyclic ring which will preferably contain 5 to 7 ring atoms, optionally 1 or 2 carbon-to-carbon double bonds and optionally one or more alkyl ($C_1$-$C_4$) or halogeno substituents on the cycloaliphatic ring.

Specific cyclopropane carboxylic acids from which the compounds I of the present invention may be structually derived include the following:-

Chrysanthemic acid, including particularly [IR, trans]- and [IR, cis]-chrysanthemic acid;

Pyrethric acid,

Dimethylcyclopropane carboxylic acid,

Tetramethylcyclopropane carboxylic acid,

2,2-Dimethyl-3-(2,2-dibromovinyl)cyclopropane carboxylic acid; particularly the (IR)-cis isomer thereo,;

2,2-Dimethyl-3-(2,2-dichlorovinyl)cyclopropane carboxylic acid,

2,2-Dimethyl-3-(1,2,2,2-tetrabromoethyl)cyclopropane carboxylic acid,

2,2-Dimethyl-3-(1,2-dibromo-2,2-dichloroethyl)cyclopropane carboxylic acid,

2,2-Dimethyl-3-(2-chloro-3,3,3-trifluoropropenyl)cyclopropane carboxylic acid or

2,2-Dimethyl-3-(tetrahydro-2-oxo-thien-3-ylidenemethyl)cyclopropane carboxylic acid.

Compounds of the invention in which $R_A$ represents a substituted cyclopropane residue of formula II can exist in the form of both geometrical and optical isomers. This is because of the unsymmetrical

substitution at $C_1$ and $C_3$ of the cyclopropane ring. Compounds of the present invention include those isomers in which the hydrogen atoms at $C_1$ and $C_3$ of the cyclopropane ring are substantially completely in the cis configuration or substantially completely in the trans configuration or mixtures thereof. The present invention also includes compounds in which the configuration at $C_1$ is substantially completely R or substantially completely S and mixtures thereof.

When $R_A$ is a group of formula II in which $R^a$ is a group of formula III in which the substitution about the ethylenic bond is asymmetrical, that is to say $R^e \neq R^f$, then the configuration of this part of the molecule can be substantially completely in the E form or substantially completely in the Z form or a mixture thereof.

$R_BCHD$ may represent the residue of any of the alcohols of formula $R_BCHDOH$ claimed or described in the specification for UK Patent No. 1413491 which give rise to significant insecticidal activity when esterified with [IR, cis]-2,2-dimethyl-3-(2,2-dibromovinyl)carboxylic acid. e.g. an alcohol claimed or described in the specification for UK Patent No. 1413491. The potency towards houseflies is usually at least 5 relative to bioresmethrin = 100 and may be 10 or more.

Typically $R_BCHD$ represents the residue of an alcohol $R_BCHDOH$ which is 3-phenoxybenzyl, 4-phenoxybenzyl, α-cyano-3-phenoxybenzyl, 4-fluoro-3-phenoxybenzyl, α-cyano-4-fluoro-3-phenoxybenzyl, 5-benzyl-3-furylmethyl, 3-benzylbenzyl, 4-benzylbenzyl, 3-benzoylbenzyl-3-phenyl-2-methylbenzyl or 3-phenyl-2-chlorobenzyl alcohol.

Compounds of formula I may be produced in accordance with a further aspect of the present invention by reaction of a compound of formula $R_ACOX$, in which X represents halogen e.g. chlorine or a substituted amide e.g. -NMeOMe with an organometallic reagent of formula $R_BCHDM$ in which M represents a metal e.g. an alkali metal or moiety comprising a metal e.g. -MgY in which Y represents halogen, preferably bromine.

In accordance with a further aspect of the present invention, a compound of formula I is produced by oxidation of an alcohol of formula $R_ACHOHCHDR_B$, prepared for example by reaction of an

aldehyde of formula $R_ACHO$ with a Grignard reagent of formula $R_BCHDMgY$, in which Y represents halogen, e.g. bromine. Oxidation of the alcohol is suitably effected by an appropriate oxidising agent such as pyridiniumchlorochromate in a good solvent such as dichloromethane.

In accordance with a further aspect of the present invention, one or more of the pesticidal compounds of formula I is formulated with an inert carrier or diluent to give a pesticidal composition.

Compositions may be in the form of dusts, granular solids, wettable powders, mosquito coils or other solid preparations or as emulsions, emulsifiable concentrates, sprays or aerosols or other liquid preparations after the addition of the appropriate solvents, diluents and surface-active agents.

The pesticidal compositions of the invention will normally contain from 0.001 to 25% by weight of the compound of formula I but the compositions can contain higher concentrations of active ingredient of formula I e.g. up to 95% for compositions to be sold as concentrates for dilution before use by the ultimate user.

The compositions of the invention can include diluents such as hydrocarbon oils, e.g. xylene or other petroleum fractions, water, anionic, cationic or non-ionic surface-active agents, anti-oxidants or other stabilisers as well as perfumes and colouring matters. These inert ingredients may be of the type and in proportions such as are conventionally used in pesticidal compositions containing pyrethroid-like compounds.

In addition to these inactive ingredients, the compositions of the present invention may contain one or more further active ingredients which may be other pesticidal compounds of the pyrethroid type or of other types and the composition may also include synergists of the type known to be capable of synergising the activity of natural pyrethrin and pyrethroid-like insecticides. Synergists of this type include piperonyl butoxide, tropital, sesamex and propyl prop-2-ynyl phenyl-phosphonate.

Compounds of formula I can be used to control pest infestation in the domestic, horticultural or agricultural or medical, including veterinary, areas.

The compounds or compositions of the invention can be used to combat pest infestation by treating pests or surfaces of environments susceptible to pest infestation with effective amounts of the active compounds of formula I suitably in compositions containing them. For example, they may be used in a domestic environment for spraying rooms to combat infestation with houseflies or other insects, they can be used for treatment of stored crops or cereals to combat infestation by insects or other pests, they can be used to spray growing crops, e.g. cotton or rice to combat infestation by common pests and they can be used in a medical or veterinary field, e.g. as a cattle spray to prevent or treat infestation by insects or other pests.

The compounds may also find application in the control of virus acquisition by and/or transmission in plants particularly when mediated by aphids such as Myzus persicae, the peach-potato aphid.

The compounds are additionally of interest for the control of pests such as the following:-

from the class of the Isopoda, for example Oniscus asellus, Armadillidium vulgare and Porcellio scaber;

from the class of the Diplopoda, for example Blaniulus guttulatus;

from the class of the Chilopoda, for example Geophilus carpophagus and Scutigera spec;

from the class of the Symphyla, for example Scutigerella immaculata;

from the order of the Thysanura, for example Lepisma saccharina;

from the order of the Collembola, for example Onychiurus armatus;

from the order of the Orthoptera, for example Blatta orientalis, Periplaneta americana, Leucophaea madarae, Blattella germanica, Acheta domesticus, Cryllotalpa spp., Locusta migratoria migratorioides, Melanoplus differentialis and Schistocerca gregaria;

from the order of the Dermaptera, for example Forficula auricularia;

from the order of the Isoptera, for example Reticulitermes spp;

from the order of the Anoplura, for example Phylloxera vastatrix, Pemphigus spp., Pediculus humanus corporis, Haematopinus spp. and Linognathus spp;

from the order of the Mallophaga, for example Trichodectes spp. and Demalinea spp;

from the order of the Thysanoptera, for example Hercinothrips fermoralis and Thrips tabaci;

from the order of the Heteroptera, for example Eurygaster spp., Dysdercus intermedius, Piesma quadrata, Cimex lectularius, Rhodnius prolius and Triatoma spp.;

from the order of the Homoptera, for example Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aondiiella aurantii, Aspidiotus hederae, Pseudococcus spp. and Psylla spp;

from the order of the Lepidoptera, for example Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Fieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima and Tortrix viridana;

from the order of the Coleoptera, for example Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestris, Atomaria spp., Oryzaephilus

surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aenus, Ptinus spp., Niptus hololeucus, Gibbium psylloides, Tribolium spp., Tenebrio molitor, Agriotes spp., Conoderus spp., Melolontha melolntha, Amphimallon solstitialis and Costelytra zealandica;

from the order of the Hymenoptera, for example Diprion spp., Hoplacampa spp., Lasius spp., Monomorium pharaonis and Vespa spp.,

from the order of the Diptera, for example Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae and Tipula paludosa;

from the order of the Siphonaptera, for example Xenopsylla cheopis and Ceratophyllus spp.;

from the class of the Arachnida, for example Scorpio maurus and Latrodectus mactans;

from the order of the Acarina, for example Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp. and Tetranychus spp.

The invention is illustrated by the following Examples.

Temperatures are in $^{o}$C and refractive indices are measured at 20$^{o}$C.

EXAMPLE 1

(IR-cis)-2,2-dimethyl-3-(2,2-dibromovinyl)-1-(1-oxo-2-(3-phenoxyphenyl)ethyl)cyclopropane

A Grignard reagent is prepared by reacting 3-phenoxybenzyl bromide (0.26 g) with magnesium turnings (24 mg) in dry ether (15 ml).

Ether is evaporated off under a stream of nitrogen and replaced by dry tetrahydrofuran (15 ml). This solution is cooled to -78°C under an atmosphere of nitrogen and a solution of (IR-cis)-2,2-dimethyl-3-(2,2-dibromovinyl) cyclopropane carboxylic acid chloride (0.3 g) in tetrahydrofuran (5 ml) is added with vigorous stirring. After 15 minutes the mixture is warmed to room temperature over 1 hour. Saturated $NH_4Cl$ solution is added and the mixture concentrated under reduced pressure. The mixture is extracted with ether (x3), washed successively with saturated $NaHCO_3$ solution, water dilute HCl, dried and the solvent removed under reduced pressure. The product is purified by thin layer chromatography on silica gel eluted with 15% ether in petroleum ether b.p. 60-80°C. Yield 0.23 g $n_D$1.5862. (Reference 6790).

EXAMPLE 2

(IR-cis-1,2-dimethyl-3-(2,2-dibromovinyl)-1-(1-oxo-2-(4-fluoro-3-phenoxyphenyl)ethyl)cyclopropane

The compound is prepared by following the procedure of Example 1, with 3-phenoxybenzyl bromide replaced by 4-fluoro-3-phenoxybenzyl bromide $n_D$1.5710. (Reference 6803).

EXAMPLE 3

(IRS-cis)-2,2-dimethyl-3-(2,2-dichlorovinyl)-1-(1-oxo-2-(3-phenoxyphenyl)ethyl)cyclopropane

A. (IRS-cis-2,2-dimethyl-3-(2,2-dichlorovinyl)cyclopropane-N-methoxy-N-methylcarboxylamide

Pyridine (0.46 g) is added to a stirred mixture of (IRS-cis-2,2-dimethyl-3-(2,2-dichlorovinyl)cyclopropane carboxylic acid chloride (0.37 g) and O,N-dimethylhydroxylamine hydrochloride (0.24 g) in dry ether (20 ml) under an atmosphere of nitrogen and cooled in an ice bath. After 0.5 hours water (2 ml) is added followed by dilute HCl and the mixture extracted with ether (x3).

Preparation of a N-methoxy-N-methylcarboxylamide by an analogous route is described in S. Nahm and S.M. Weinrebs, Tetrahedron Lett. 1981, 22 (39) p.3815-8.

The combined extracts are washed with saturated $NaHCO_3$, water dried and the solvent removed under reduced pressure. Yield 0.3 g, $n_D$1.5090.

B. (IRS- cis)-2,2-dimethyl-3-(2,2-dichlorovinyl)-1-(1-oxo-2-(3-phenoxyphenyl)ethyl)cyclopropane

A Grignard reagent is prepared by reacting 3-phenoxybenzyl bromide (0.34 g) with magnesium (31 mg) in dry ether (20 ml). Ether is evaporated off under a stream of nitrogen and replaced by dry tetrahydrofuran (20 ml). The solution is cooled to 0°C under an atmosphere of nitrogen, and a solution of (IRS- cis)-2,2-dimethyl-3-(2,2-dichlorovinyl)cyclopropane N-methoxy-N-methyl carboxylamide (0.22 g) in tetrahydrofuran (5 ml) is added in one portion with vigorous stirring. After 1.5 hours ca. 10% HCl dissolved in ethanol (5 ml) is added, the mixture stirred for 10 minutes, poured onto water and extracted with ether (x3). The ether is washed with water, dried and the solvent evaporated off under reduced pressure. The product is purified by thin layer chromatography on silica gel eluted with 15% ether in petroleum ether b.p. 60-80°C. Yield 150 mg $n_D$1.5748. (Reference 6818).

EXAMPLE 4

(IRS- trans)-2,2-dimethyl-3-(2,2-dichlorovinyl)-1-(1-oxo-2-(3-phenoxyphenyl)ethyl)cyclopropane

(IRS- trans)-2,2-dimethyl-3-(2,2-dichlorovinyl)cyclopropane-N-methoxy-N-methyl carboxylamide is prepared by following the procedure for the corresponding (IRS- cis) amide in Example 3, in which the (IRS- cis)-2,2-dimethyl-3-(2,2-dichlorovinyl)cyclopropane acid chloride is replaced by the corresponding (IRS- trans) acid chloride. The product has m.p. 68-69°C.

The title compound of the present Example is then prepared by following the procedure of Example 3 replacing the (IRS- cis) amide by the above (IRS- trans) amide and has $n_D$1.5730. (Reference 6831).

Pesticidal activity is assessed against houseflies and mustard beetles by using the following techniques:-

EXAMPLE 5

(IRS- cis/trans)-2,2-dimethyl-3-(2,2-dichlorovinyl)-1-(1-oxo-2-(3-benzoylphenyl)ethyl)cyclopropane

A solution of 3-benzoylbenzyl bromide (0.26g) and (IRS- cis/trans)-2,2-dimethyl-3-(2,2-dichlorovinyl) cyclopropane acid chloride (0.23g) in dry 1,2 dimethoxyethane (2ml) is added to a stirred suspension of activated zinc powder (0.13g) and bis (triphenylphosphine)palladium(II) chloride (40mg) in 1,2-dimethoxyethane(1 ml) under an atmosphere of nitrogen at room temperature (c.f. reference T. Sato, K. Naruse, M. Enokiya and T. Fugisaura, Chem. Lett. 1981, p.1135-1138). After 1 hour, 2NHCl (10ml) is added followed by ether (15ml). The mixture is stirred for 10 minutes, extracted with ether (x3), washed with water, saturated $NaHCO_3$, stirred and the solvent evaporated under reduced pressure. The product is purified by thin layer chromotography on silica eluted with 10% ether in petroleum ether b.p. 60-80. Yield 0.2g, $n_D$1.5572 (Reference 7012).

Houseflies (Musca domestica)

Female flies are treated on the thorax with a one microlitre drop of insecticide dissolved in acetone. Two replicates of 15 flies are used at each dose rate and 6 dose rates are used per compound under test. After treatment, the flies are maintained at a temperature of $20^o \pm 1^o$ and kill is assessed 24 and 48 hours after treatment. $LD_{50}$ values are calculated in micrograms of insecticide per fly and relative toxicities are calculated from the inverse ratios of the $LD_{50}$ values (see Sawicki et al, Bulletin of the World Health Organisation, 35, 893, (1966) and Sawicki et al, Entomologia and Exp. Appli. 10 253, (1967)).

Mustard beetles (Phaedon cochleariae Fab)

Acetone solutions of the test compound are applied ventrally to adult mustard beetles using a micro drop applicator. The treated insects are maintained for 48 hours after which time kill is assessed. Two replicates of 40 to 50 mustard beetles are used at each dose level and 5 dose levels are used for each compound.

$LD_{50}$ values and thence relative potencies are calculated as for houseflies.

For both insect species relative potencies are calculated by

comparison with 5-benzyl-3-furylmethyl (IR)-*trans*-chrysanthemate (Bioresmethrin) which is one of the more toxic chrysanthemate esters known to houseflies and mustard beetle, its toxicity being about 24 times that of allethrin to houseflies and 65 times that of allethrin to mustard beetles.

Results

Relative potencies to Houseflies and Mustard Beetles (Bioresmethrin = 100) are given under HF and MB respectively in the Table.

0171249



TABLE

COMPOUNDS I: $R_A$ = 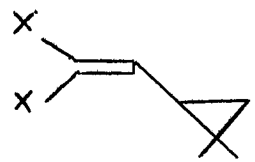 D in $-CHDR_B$ = H

| Example | Reference Number | X | Isomer | $R_B$* | HF | MB |
|---|---|---|---|---|---|---|
| 1 | 6790 | Br | IR- cis | 3POP | 6.9 | 16 |
| 2 | 6803 | Br | IR- cis | 4F-3POP | 16 | 36 |
| 3 | 6818 | Cl | IRS- cis | 3POP | 29 | 9.6 |
| 4 | 6831 | Cl | IRS- trans | 3POP | 15 | 5.5 |
| 5 | 7012 | Cl | IRS- cis/trans | 3BP | 3.2 | 3.3 |

[a]Bioresmethrin = 100

*3-POP represents: 3-phenoxyphenyl, 4F-3POP: 4-fluorophenoxyphenyl and 3-BP: 3-benzoylphenyl

CLAIMS

1. A compound of formula I

I $R_ACOCHDR_B$

in which formula:-

$R_A$ represents a group $ArCR_1R_2$ - in which Ar represents a phenyl or naphthyl group optionally substituted by one or more halogen, haloalkoxy methylenedioxy or $C_1$-$C_6$ alkyl groups;

$R_1$ representing methyl, ethyl, isopropyl or cycloalkyl;

$R_2$ representing hydrogen or methyl; or

$R_1$ and $R_2$ together with the carbon to which they are attached representing jointly a cycloalkyl group optionally carrying one or more halogen substituents;

or $R_A$ represents the residue of a pyrethroidal acid $R_ACO_2H$ the 5-benzyl-3-furylmethyl ester of which is significantly insecticidal;

$R_B$ represents the residue of an alcohol $R_BCHDOH$ in which D is hydrogen or cyano and of which the [1R, cis] 2,2-dimethyl-3-(2,2-dibromovinyl)cyclopropane carboxylic ester is significantly insecticidal, provided that when the alcohol is of formula II:

II 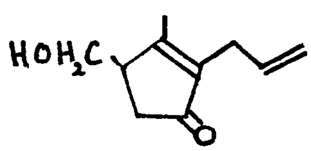

$R_A$ is other than the residue of chrysanthemic acid.

2. A compound according to Claim 1 in which $R_A$ represents the residue of a pyrethroidal acid $R_ACO_2H$.

3. A compound according to Claim 2 in which the acid is such that the 5-benzyl-3-furylmethyl ester thereof shows a potency of at least 5 relative to bioresmethrin = 100 towards houseflies.

4. A compound according to any preceding claim in which $R_A$ represents the residue of one of the following acids:

Chrysanthemic acid, Pyrethric acid, Dimethylcyclopropane carboxylic acid, Tetramethylcyclopropane carboxylic acid, 2,2-dimethyl-3-(2,2-dibromovinyl)cyclopropane carboxylic acid, 2,2-dimethyl-3-(2,2-dichlorovinyl)cyclopropane carboxylic acid 2,2-Dimethyl-3-(1,2,2,2-tetrabromoethyl)cyclopropane carboxylic acid, 2,2-Dimethyl-3-(1,2-dibromo-2,2-dichloroethyl)cyclopropane carboxylic acid

2,2-Dimethyl-3-(2-chloro-3,3,3-trifluoropropenyl)cyclopropane carboxylic acid or

2,2-Dimethyl-3-(tetrahydro-2-oxo-thien-3-ylidenemethyl)cyclopropane carboxylic acid.

5. A compound according to any preceding claim in which $R_B$CHD represent the residue of any alcohols of formula $R_B$CHDOH which give rise to significant insecticidal activity when esterified with [IR, cis] -2,2-dimethyl-3-(2,2-dibromovinyl)carboxylic acid.

6. A compound according to Claim 5 in which the alcohol is such that the [IR, cis] -2,2-dimethyl-3-(2,2-dibromovinyl) carboxylic ester thereof has a potency towards houseflies which is at least 5 relative to bioresmethrin = 100.

7. A compound according to Claim 5 or 6 in which $R_B$CHD represents the residue of an alcohol $R_B$CHDOH which is 3-phenoxybenzyl, 4-phenoxybenzyl, α-cyano-3-phenoxybenzyl, 4-fluoro-3-phenoxybenzyl, α-cyano-4-fluoro-3-phenoxybenzyl, 5-benzyl-3-furylmethyl, 3-benzylbenzyl, 4-benzylbenzyl, 3-phenyl-2-methylbenzyl or 3-phenyl-2-chlorobenzyl alcohol.

8. A compound of formula I substantially as described in any one of the Examples.

9. A process for the production of a compound of formula I, in which a compound of formula $R_ACH_2COX$, wherein X represents halogen or a substituted amide is reacted with an organometallic reagent of formula $R_B$CHDM, in which formula M represents a metal or moiety comprising a metal.

10. A process for the production of a compound of formula I in which a compound of formula: $R_ACH_2$CHOH $CHDR_B$ is oxidised.

11. Intermediates useful in the production of compounds of Formula

I of formula $R_ACH_2COX$, X representing halogen or a substituted amide or of formula $R_ACH_2CHOH\ CHDR_B$.

12. A pesticidal composition comprising a compound of formula I formulated with an inert carrier or diluent.

13. A method of combatting a pest infestation which comprises treating a pest or an environment susceptible to pest infestation with an effective amount of a compound of formula I.